**Europäisches Patentamt**

**European Patent Office**

**Office européen des brevets**

⑩

(11) Publication number: **0 188 309**
**B1**

(12) # EUROPEAN PATENT SPECIFICATION

(45) Date of publication of patent specification: **23.05.90**

(21) Application number: **86300021.2**

(22) Date of filing: **03.01.86**

(51) Int. Cl.⁵: **A 61 K 9/50,** C 12 N 11/04, A 61 L 27/00, A 61 K 35/12, A 61 F 2/02 // C12P21/00, C12N5/00

(54) Microencapsulation of living cells.

(30) Priority: **03.01.85 GB 8500121**

(43) Date of publication of application:
**23.07.86 Bulletin 86/30**

(45) Publication of the grant of the patent:
**23.05.90 Bulletin 90/21**

(84) Designated Contracting States:
**AT BE CH DE FR GB IT LI LU NL SE**

(56) References cited:
FR-A-2 453 642
GB-A-2 094 750
US-A-4 487 758

(73) Proprietor: **CONNAUGHT LABORATORIES LIMITED**
**1755 Steeles Avenue West**
**Willowdale Ontario M2N 5T8 (CA)**

(72) Inventor: **Sun, Anthony Mein-Fang**
**4 Barkwood Crescent**
**Willowdale Ontario M2H 3G6 (CA)**
Inventor: **Marzakhan-Gharapetian, Hrire**
**623 Finch Avenue West Apt. 612**
**Willowdale Ontario M5R 3V4 (CA)**

(74) Representative: **Burford, Anthony Frederick et al**
**W.H. Beck, Greener & Co. 7 Stone Buildings**
**Lincoln's Inn**
**London WC2A 3SZ (GB)**

Courier Press, Leamington Spa, England.

## Description

The present invention relates to the microencapsulation of living cells, within new microporous, biocompatible, semi-permeable membranes.

The microencapsulation of living cells has been employed for a number of years in attempts to create artificial organs for human use, that are not subject to the rejection phenomena that occurs with normal tissue and unencapsulated materials. The process has been the subject of a number of issued patents, for example, U.S. Patents Nos. 4,409,331 and 4,322,311, and a number of pending patent applications.

The major direction of the recent work in this area has been to obtain a membrane that is completely biocompatible, possesses the required strength to retain the shape and integrity of the microcapsules, and whose micropore size can be controlled to the extent of allowing essential nutrients to pass freely from the environment to cell and required products, for example, insulin, to go from cell to environment, without allowing larger and possibly toxic materials to pass.

For example, in published European Patent Publications Nos. 0127713 A2 and 0127989 A2, there is described the formation of a semi-permeable membrane which is both biocompatible and yet is able to protect transplanted tissue and cells from destruction by the body immune system, such that, in animal tests, a single intraperitoneal transplant of encapsulated islets reversed the diabetic state for more than one year.

In much of the previous work and in the aforementioned patents and published applications, cells are suspended in a polyanionic polysaccharide gell and the required membrane is formed around this polyanionic polysaccharide containing the cells. In the practical application of the procedure, alginic acid or a similar polyanionic polysaccharide is first converted to the insoluble gelled calcium salt, which permits the formation of a membrane around the suspended cells. In the aforementioned published patent applications, the membrane formed on the gelled polyanionic polysaccharide has been polylysine, which is biocompatible and gives microcapsules which can survive in vivo for periods up to a year.

After the formation of the membrane to give the microencapsulated cells, the insoluble calcium gel is often converted back to the soluble form of the polyanionic polysaccharide by suspending or washing the microcapsules in physiological saline.

There have been indications that membrane polymers obtained from monomers that were not of natural origin, such as ethyleneimine, were not biocompatible and led to rejection of the microcapsules by the in vivo system.

To date there has been no suggestion that materials other than gels of natural origin may provide a biocompatible entrapment medium for the cells about which a membrane could be formed without damage to the viability of the cells. It has also appeared that the membrane polymer should comprise a material of biological origin.

In accordance with the present invention, it has now been surprisingly found that living cellular material can be microencapsulated in a system where certain polymers formed from acrylic acid derivatives may be used to form a matrix for the cellular material about which a semi-permeable membrane, also consisting of polymers of acrylic acid ester derivatives, may be formed. In general, in this description of the invention, the polymers that are used to form the matrix are polyanionic whereas those forming the membranes are polycationic. However, it is also within the scope of the invention for the matrix polymer to be polycationic and the membrane to be polyanionic.

The present invention also includes a method of forming microcapsules, wherein a suspension of living cells in an aqueous solution of a polyanionic acrylic polymer is introduced in droplet form to an aqueous solution of a polycationic acrylic polymer, causing an interaction between the anionic and cationic groups at the interface between the suspension of living matter and the polymer solution. As a result, a water-insoluble polymer-polymer complex is formed, thus forming a microcapsule enclosing the living material suspension. In some instances, it may be possible to reverse the polymer reactants, and form the suspension of the living tissue in the polycationic polymer and introduce droplets thereof to an aqueous solution of the polyanionic acrylic polymer.

Once the initial microcapsules are formed from the droplets, they may be further reacted with an additional polymer containing reactive groups to increase the thickness of the microporous semi-permeable membrane wall and to provide the required strength to retain the shape and integrity of the microcapsules and in addition control the porosity of the polymer capsule.

It is preferred to provide an outer coating of a polymer containing group reactive with the polycationic and/or polyanionic groups of the membrane polymer, typically epoxy groups, so as to be covalently bound to the membrane polymer and thereby provide the desired structural integrity and strength.

The microcapsules may have any desired diameter consistent with the nature of the cellular material and the end use of the microcapsules. Generally, the diameter ranges from about 150 to about 2000 microns and preferably, for cardiovascular injection, the microcapsules have a diameter from about 150 to about 500 microns.

The term "living cellular material" is used herein to refer to a variety of forms of living matter, including tissue, individual cells, multicellular fractions and other biologically-active material. The invention has wide application to a variety of living cellular material, even though only two specific embodiments of such living cellular material, namely Islets of Langerhans and hybridoma cells, are described in detail. It will be apparent to a person skilled in the art, that the general teachings of membrane formation are broadly applicable to all living cellular material.

The interaction of the polyanionic and polycationic groups in the two polymeric acrylic acid derivatives is effective to form a biocompatible microporous semi-permeable membrane about living cellular material without adversely affecting the viability of the living cellular material. Once encapsulated, the living cellular material remain viable, healthy and physiologically-active and are capable of on-going metabolism.

By the utilisation of the interaction of polyanionic groups in an acrylic polymer and cationic groups on another polymer, there is able to be produced for the first time, encapsulated living cellular material, including Islets of Langerhans and hybridoma cells, using synthetic polymeric materials rather than natural-source polyanionic polysaccharides. An advantage of using synthetic rather than natural polymers for microencapsulation of living cellular materials is the ability to design and prepare a large number of copolymers having a wide variety of structures and properties, enabling microcapsules to be developed to suit specific applications. Further, the necessity of gelation and subsequent reliquification steps, as required in the prior art procedures described above, is eliminated, thereby simplifying the microencapsulation procedure.

The term "acrylic polymer" is used herein to refer to polymeric materials which are homopolymers or copolymers of various unsaturated acrylic acid or methacrylic acid monomers, at least one of which contains the anionic or cationic group(s) desired in the polymer. These polymers usually are insoluble but may be solubilized as aqueous solutions for use herein by providing the same in salt form by the use of acid or base, as appropriate. Others of such polymers are per se water-soluble and do not require separate solubilization for use herein.

A description of certain preferred aspects and embodiments of the invention now follows. This following description is not to be construed as limiting the generality of the invention, as described above and as defined in the appended claims.

In the present invention, living cellular material is encapsulated within a biocompatible semi-permeable membrane, which takes the form of a cross-linked hydrogel. The material to be encapsulated is suspended in a physiologically-compatible medium containing a water-soluble polyanionic acrylic polymer. The medium containing the polymer and the tissue to be encapsulated are formed into droplets which are introduced into an aqueous solution of a water-soluble polycationic acrylic polymer. An interaction takes place between the anionic and cationic groups in the two acrylic polymers at the interface between the suspension of living matter and the second acrylic polymer solution, thus forming a water-insoluble polymer-polymer complex as the initial layer of the microcapsule.

The droplets of suspension of living cellular material may be formed in any convenient manner. It is preferred to employ the electrostatic droplet generation procedure described in copending United States patent application Serial No. 631,471 filed July 16, 1984, assigned to the applicant herein, the disclosure of which is incorporated herein by reference. This procedure is preferred in view of the very small diameter spherical droplets which may be formed thereby and the control that can be exercised over the droplet-forming operation, as described in detail in the aforementioned copending United States patent application.

In the electrostatic droplet-forming procedure, the aqueous suspension is extruded downwardly at a first location and the extruded material is charged with a charge of one polarity. A charge of opposite polarity is established at a second location spaced vertically below the first location. A difference in voltage is provided between the two locations to form a liquid droplet from the extruded material and draw the same towards the second location.

In one embodiment of this procedure, the first location comprises an axially-downwardly directed electroconductive needle, and the second location comprises the aqueous solution of the second acrylic polymer. In another embodiment of this procedure, the first location comprises an axially-downwardly directed electroconductive needle, the second location comprises a ring of electroconductive material surrounding and concentric with the axis of the needle, and the aqueous solution of the second acrylic polymer is located below the second location to receive and collect the formed droplets therein.

The voltage difference for droplet formation is preferably applied cyclically in pulses to continuously-extruded droplet-forming aqueous suspension to effect continuous production of droplets for microencapsulation.

The monomers used in forming the acrylic copolymers employed in this invention must be of high purity. For synthesis of the polymers used in the experiments described herein, the monomers were purified by triple distillation and the copolymers synthesized by free radical solution polymerisation in ampoules sealed under vacuum. They were then purified by precipitation and dried. The copolymers were solubilised in aqueous medium as their salts by the addition of either acid or base, brought to approximately neutral pH and sterilised, either by autoclaving or filtration through a 0.22 micron filter. Any other convenient preparation and purification procedure may be adopted.

A variety of types of polycationic acrylic polymer containing cationic groups may be used in the initial reaction with the polyanionic acrylic copolymer (copolymer A) to form the microcapsules and to obtain the microcapsule formation.

Among the types that may be used are:

(a) a polycationic acrylic copolymer containing tertiary amine groups and carboxylic acid groups (copolymer B),

(b) a polycationic acrylic copolymer containing primary and/or secondary amine groups and tertiary amine groups (copolymer C), and

(c) a polycationic acrylic copolymer containing epoxy groups and tertiary amine groups (copolymer D).

Copolymers B may be formed from a mixture of monomers capable of reacting to form a polycationic acrylic copolymer containing tertiary amine and carboxylic acid groups, for example, dialkylaminoacrylates and/or methacrylates, along with small quantities of acrylic and/or methacrylic acid.

Polymer C may be formed from a mixture of monomers capable of reacting to form a polycationic acrylic copolymer containing primary and/or secondary amine groups, for example, amino alkyl acrylates and/or methacrylates and mono and/or dialkyl acrylates and/or methacrylates, alkyl acrylates and/or methacrylates, and hydroxyalkyl methacrylates and or methacrylates.

Polymer D may be formed from a mixture of monomers capable of reacting to form an acrylic copolymer containing epoxy functionality and/or tertiary amine groups, for example, alkyl acrylates and/or methacrylates, hydroxyacrylates and/or methacrylates, alkylamides and/or methacrylamides and/or vinyl pyrollidone, and epoxyalkyl acrylates and/or methacrylates.

A wide variety of polycationic copolymers (copolymers B, C and D) may be employed based on copolymerized combinations of 2-hydroxyethylmethacrylate (HEMA), dimethylaminoethylmethacrylate (DMAEMA), diethylaminoethylacrylate (DEAEA) t-butylaminoethylmethacrylate (t-BAEMA), 2,3-epoxypropylmethacrylate (PMA), diethylaminoethylmethacrylate (DEAEMA), ethylmethacrylate (EMA) and MA or AA.

A wide variety of polyanionic copolymers (copolymer A) may be employed based on copolymerized combinations of 2,3-dihydroxypropylmethacrylate (HPMA), 2-hydroxyethylmethacrylate (HEMA), methacrylic acid (MA) or acrylic acid (AA) and methylmethacrylate (MMA) or ethylmethacrylate (EMA) or a combination of both.

Thicker, stronger and smoother membranes are formed when the molar percentage of the cationic or anionic monomers in the comonomer feed composition is 30 to 50%.

As noted earlier, once the microcapsules are formed by ionic interaction between the acrylic polymers, the thickness of the membrane, along with other properties, such as porosity, may be increased by applying an additional polymer layer (copolymer E). The nature of the polymer used depends to some extent on the order of the reactants used in the initial membrane forming step and the nature of the polycationic polymer employed.

For example, where droplets of the aqueous suspension of the living cellular material in polyanionic acrylic polymer are introduced to an aqueous solution of polymer B, the microcapsules so formed may be transferred into a solution of an additional polycationic polymer B or C, which induces the formation of a second layer of polyelectrolyte complex, formed as a result of interactions between the groups on the polymers.

Where droplets of the aqueous suspension of the living cells in polyanionic acrylic polymer are introduced to an aqueous solution of polymer C or an additional layer has been formed on the microcapsule using polymer C, the microcapsules so formed may be transferred into an aqueous solution of an acrylic polymer containing reactive epoxy groups (polymer D), or similar reactive groups. A chemically-bound layer is formed on the surface of the microcapsule as a result of reaction between the epoxy group in polymer D with the amine, hydroxy and/or carboxylate functions present on the membranes of the microcapsules formed in the solution of polymer C.

In order to neutralize any possibly remaining positive charges on the capsule membranes, the capsules prepared by any of the above methods preferably are placed in an aqueous solution of polymer A.

Following formation of the microcapsules of the desired physical characteristics using the above-noted procedures, the microcapsules may be washed in a suitable aqueous solution to remove excess reactants, prior to placing them in a physiological medium. One suitable washing procedure is to wash the microcapsules three times in Hank's salt solution.

The process of the invention is useful for microencapsulation of living cellular material, which may be in the form of living or viable tissue, multicellular fractions or individual cells. The membrane surrounding the cells is biocompatible so that the cells remain viable, physiologically active and are capable of ongoing metabolism. The membrane is microporous and semipermeable, permitting essential nutrients to pass freely from the environment of the microcapsules to the cells and the metabolic products to pass from the cells to the environment of the microcapsules.

The microcapsules may be used for in vitro production of production of metabolites from the encapsulated cells, but are particularly useful for in vivo implantation or injection to create artificial organs for human use in the treatment of mammalian disorders. In one particular embodiment of the invention, Islets of Langerhans may be microencapsulated using the process of the invention and the microcapsules implanted to act as a source of insulin to a diabetic animal, including humans. A further application is the microencapsulation of liver cells to provide a temporary organ for animals, including humans, suffering liver damage. Cells performing other functions also may be microencapsulated using the procedure of the invention, for example, hybridoma cells and the resulting products, for example, antibodies, may be isolated by rupture of the membrane.

The invention is illustrated by the following Examples:

A series of experiments was carried out to effect microencapsulation of hybridoma cells and Islets of Langerhans. In the case of hybridoma cells, they were derived from BALB/c mice immunised cells with the NS-1 murine plasmacytoma cell line. These hybridomas were normally grown in a modified RPMI-1640 medium.

The microcapsules were formed by suspending the hybridoma cells (M2) in a 1.5% (w/v) solution of the copolymer A in RPMI medium and using a droplet generator in combination with syringe pump extrusion to introduce the droplets into a 3.9% aqueous solution of the copolymer B. The resulting microcapsules were washed and then coated with a thin layer of the copolymer C, by treating the capsules with a 3.9% (w/v) aqueous solution of this copolymer. The capsules were washed and further strengthened by treatment with a 0.1% (w/v) solution of the copolymer D. This layer of membrane was covalently bound through the epoxy groups rather than ionically bound as are the previous copolymer layers.

The approximate size of the microcapsules was 400 microns and each capsule initially contained less than 10 hybridoma cells. The cells multiplied and gradually filled the microcapsules over a period of eight days. The final cell density was $5 \times 10^8$ cells/ml after eight days of growth. Immunoglobulin G was produced by the microencapsulated cells and after the eighth day the concentration was shown to be 3 ug/ml.

In the case of Islets of Langerhans, the cells obtained from an animal pancreas, were microencapsulated by suspending them in CMRL 1969 medium containing copolymer A and forming droplets which went into a solution of copolymer B. The capsules were strengthened by formation of another polymer layer by placing in copolymer C. The Islets were shown to remain viable and produce insulin for at least two weeks.

In the series of experiments, the copolymer A was prepared from 26.8 mole per cent HEMA, 32.8 mole per cent AA and 40.4 mole per cent MMA monomers; the copolymer B was prepared from 7.5 mole per cent HEMA, 17.6 mole per cent DMAEMA, 17.5 mole per cent DEAEA, 53.3 mole per cent EMA and 4.1 mole per cent AA; the copolymer C was prepared from 7.8 mole per cent HEMA, 19.3 mole per cent DMAEMA, 18.5 mole per cent t-butylaminoethylmethacrylate (tBAEMA) and 54.5 mole per cent EMA; and the copolymer D was prepared from 5.0 mole per cent 2,3-epoxypropylmethacrylate (EPMA), 39.9 mole per cent diethylaminoethylmethacrylate (DEAEMA) and 55.2 mole per cent of EMA.

The following specific examples illustrate the invention and provide specific details of the series of experiments referred to above.

Example 1

This Example illustrates the preparation of copolymer A.

The following reactants were added to 25 ml of methanol and 1 ml of water: 0.8 ml of AA, 1.54 ml of MMA and 1.20 ml HEMA monomers. Potassium persulphate (2.2 mg) was added and the mixture was heated at 60°C for 80 hours. The polymer was isolated by precipitation, and purified by further precipitation.

Example 2

This Example illustrates the preparation of copolymer B.

Copolymer B was polymerised by adding to a mixture of 10 ml of toluene and 9.5 ml of methanol, 1.25 ml of DMEAMA, 1.4 ml of DEAEA, 0.4 ml HEMA, 2.4 ml EMA monomers with 420 mg of AMIB as the initiator. The polymerisation was performed at 50°C for 80 hours, and the product was isolated and purified by precipitation.

Example 3

This Example illustrates the preparation of copolymer C.

Copolymer C was synthesized by polymerisation of 1.35 ml DMAEMA, 1.6 ml of tBAEMA, 0.4 ml of HEMA and 2.8 ml of EMA in 10 ml of toluene and 8.5 of methanol as solvents and using 420 mg of AMIB as an initiator. The polymerisation was performed at 50°C for 24 hours, and the product was isolated and purified by precipitation.

Example 4

This Example illustrates the preparation of copolymer D.

Copolymer D was synthesized by polymerisation of 0.28 ml of 2,3-epoxypropylmethacrylate (EPMA), 3.4 ml DEAEMA and 2.85 ml EMA using 420 mg of AMIB as an initiator in a mixture of 10 ml of toluene and 8.5 ml of methanol as solvents. The polymerisation was performed at 50°C for 29 hours. The product was isolated and purified by precipitation.

Example 5

This Example illustrates the preparation of hybridoma cells for microencapsulation.

Hybridoma cells were derived from a fusion of BALB/c mouse spleen cells immunised with a human IgM kappa paraprotein and the NS-1 plasmacytoma cell line. The resultant hybrids were screened for reactivity to pooled normal IgM. Subclones were subsequently propagated in RPMI-1640 supplemented with 10% (v/v) fetal calf serum (FCS), 100 u/ml penicillin, 100 ug/ml streptomycin, 2 mM glutamine, 2 mg/ml sodium bicarbonate and cultured at 37°C in a humidified incubator with 5% $CO_2$ in air. The supplemented medium is designated RPMI.

Example 6

This Example illustrates microencapsulation of hybridoma cells.

Cell microencapsulation was performed using a suspension of hybridoma cells in a 1.5% (w/v) solution of the copolymer A in RPMI. Droplets were generated using the electrostatic droplet generator described in the aforementioned U.S. patent application Serial No. 631,471 and introduced into a 3.9% (w/v) aqueous solution of the copolymer B to form a capsule membrane. The microcapsules were washed three times with modified Hank's buffered salt solution (HBSS) and coated with a thin layer of the polycationic copolymer C, by treating the microcapsules with a 3.9% (w/v) aqueous solution of the latter polymer. The microcapsules were washed three times with HBSS and strengthened by further treatment with a 0.1% (w/v) solution of the copolymer D. The microcapsules were then cultured in RPMI, in a humidified incubator at 37°C with 5% $CO_2$ in air.

Example 7

This Example illustrates the microencapsulation of Islets of Langerhans.

Microencapsulation of Islets of Langerhans was performed in a similar manner. Approximately 400 Islets of animal origin were suspended in 1 ml of the medium CMRL-1969 at twice its normal concentration and containing 10% fetal calf serum. The suspension was diluted with an equal volume of a 3.3% (w/v) aqueous solution of copolymer A and droplets were formed and encapsulated in copolymer B as before. The microcapsules were further washed and then strengthened by additional immersion in copolymer D. The Islets were shown to be viable by the use of Trypan blue stain. In addition the encapsulated Islets were shown by microassay to be still producing insulin after ten days in the medium.

In summary of this disclosure, the present invention provides a novel procedure for the microencapsulation of living cellular material using acrylic polymers.

**Claims**

1. A microcapsule comprising a biocompatible semipermeable membrane surrounding a core containing living cellular material, characterized in that said membrane is formed by the ionic interaction of a matrix biocompatible, non-toxic acrylic polymer which is polyanionic or polycationic and a membrane biocompatible, non-toxic acrylic polymer which is oppositely charged to said matrix acrylic polymer.

2. A microcapsule as claimed in Claim 1, characterized in that said matrix acrylic polymer is polyanionic and said membrane acrylic polymer is polycationic.

3. A microcapsule as claimed in Claim 1 or Claim 2, having a diameter of 150 to 2000 micrometres.

4. A microcapsule as claimed in any one of Claims 1 to 3, characterized in that said membrane further comprises at least one additional layer of biocompatible polymeric material.

5. A microcapsule as claimed in Claim 4, characterized in that said at least one additional layer includes an outer biocompatible acrylic polymer material containing groups covalently bonded to a subsurface layer of said membrane.

6. A microcapsule as claimed in any one of Claims 1 to 5, characterized in that said matrix acrylic polymer is polyanionic and in that said membrane polymer is polycationic and contains (a) tertiary amine groups and carboxylic acid groups, (b) primary and/or secondary amine groups, or (c) epoxy groups and/or tertiary amine groups.

7. A microcapsule as claimed in any one of Claims 1 to 6, characterized in that said cellular material is hybridoma cells or Islets of Langerhans.

8. A method for the formation of microcapsules, characterized by (a) forming an aqueous suspension of living cellular material in an aqueous solution of a first, water-soluble biocompatible, non-toxic acrylic polymer which is polyanionic or polycationic, and (b) introducing said aqueous suspension in droplet form to an aqueous solution of a second water-soluble, biocompatible, non-toxic acrylic polymer which is oppositely charged to said first acrylic polymer, so as to cause an interaction between the anionic and cationic groups at the interface between the suspension of living matter and the second acrylic polymer solution and the formation of a water-insoluble polymer-polymer complex.

9. A method as claimed in Claim 8, characterized in that said microcapsules are reacted with an additional polymer containing groups reactive with surface groups of the microcapsule to increase the thickness of the microporous membrane wall and to provide sufficient strength to retain a spherical shape and structural integrity upon in vivo introduction.

10. A method as claimed in Claim 8 or Claim 9, characterized in that said aqueous suspension and said aqueous solution both have an approximately neutral pH and said first and second acrylic polymers are present in solubilized salt form.

**Patentansprüche**

1. Mikrokapsel, umfassend eine biokompatible semipermeable Membran, welche einen Kern umgibt, der lebendes zelluläres Material enthält, dadurch gekennzeichnet, daß die Membran durch ionische Wechselwirkung eines biokompatiblen, ungiftigen Acrylmatrixpolymers, welches polyanionisch oder polykationisch ist und eines biokompatiblen, ungiftigen Acrylmembranpolymers gebildet wird, welches gegenüber dem Acrylmatrixpolymer entgegengesetzt geladen ist.

2. Mikrokapsel nach Anspruch 1, dadurch gekennzeichnet, daß das Matrixacrylpolymer polyanionisch ist und das Membranacrylpolymer polykationisch ist.

3. Mikrokapsel nach Anspruch 1 oder 2 mit einem Durchmesser von 150 bis 2000 um.

4. Mikrokapsel nach einem der Ansprüche 1 bis 3, dadurch gekennzeichnet, daß die Membran zusätzlich mindestens eine zusätzliche Schicht eines biokompatiblen polymeren Materials umfaßt.

5. Mikrokapsel nach Anspruch 4, dadurch gekennzeichnet, daß die mindestens einmal ausgebildete zusätzliche Schicht ein äußeres biokompatibles Acrylpolymermaterial umfaßt, welches Gruppen enthält, die kovalent an eine unterhalb der Oberfläche liegende Schicht der Membran gebunden sind.

6. Mikrokapsel nach einem der Ansprüche 1 bis 5, dadurch gekennzeichnet, daß das Matrixacrylpolymer polyanionisch ist und daß das Membranpolymer polykationisch ist und (a) tertiäre Amingruppen und Carbonsäuregruppen, (b) primäre und/oder sekundäre Amingruppen, oder (c) Epoxygruppen und/oder tertiäre Amingruppen enthält.

7. Mikrokapsel nach einem der Ansprüche 1 bis 6, dadurch gekennzeichnet, daß es sich bei dem zellulären Material um Hybridomazellen oder Langerhans'sche Inseln handelt.

8. Verfahren zur Herstellung von Mikrokapseln, gekennzeichnet durch (a) Herstellung einer wässrigen Suspension von lebendem zellulären Material in einer wässrigen Lösung eines ersten, wasserlöslichen, biokompatiblen ungiftigen Acrylpolymers, welches polyanionisch oder polykationisch ist und (b) Einführung dieser wässrigen Suspension in Tröpfchenform in eine wässrige Lösung eines zweiten, wasserlöslichen, biokompatiblen, ungiftigen Acrylpolymers, welches gegenüber dem ersten Acrylpolymer entgegengesetzt geladen ist, so daß eine Wechselwirkung zwischen den anionischen und kationischen Gruppen an der Zwischenfläche zwischen der Suspension aus lebendem Material und der zweiten Acrylpolymerlösung und die Bildung eines wasserunlöslichen Polymer-Polymerkomplexes herbeigefügt wird.

9. Verfahren nach Anspruch 8, dadurch gekennzeichnet, daß die Mikrokapseln mit einem zusätzlichen Polymer zur Reaktion gebracht werden, welches Gruppen enthält, die gegenüber den Oberflächengruppen der Mikrokapseln reaktiv sind, um die Dicke der mikroporösen Membranwandung zu erhöhen und um eine ausreichende Stärke für die Beibehaltung einer kugelförmigen Form und einer strukturellen Unversehrtheit bei einer in-vivo-Verabreichung zu erhalten.

10. Verfahren nach einem der Ansprüche 8 oder 9, dadurch gekennzeichnet, daß die wässrige Suspension und die wässrige Lösung einen nahezu neutralen pH-Wert aufweisen und das erste und das zweite Acrylpolymer in gelöster Salzform vorliegen.

**Revendications**

1. Microcapsule comprenant une membrane semi-perméable biocompatible entourant un noyau contenant un matériau cellulaire vivant, caractérisée en ce que ladite membrane est formée par l'interaction ionique d'un polymère acrylique de matrice, biocompatible et non toxique, qui est polyanionique ou polycationique et d'un polymère acrylique de membrane, biocompatible et non toxique, qui a une charge opposée à celle dudit polymère acrylique de matrice.

2. Microcapsule selon la revendication 1, caractérisée en ce que ledit polymère acrylique de matrice est polyanionique et ledit polymère acrylique de membrane est polycationique.

3. Microcapsule selon la revendication 1 ou la revendication 2 ayant un diamètre de 150 à 2000 micromètres.

4. Microcapsule selon l'une quelconque des revendications 1 à 3, caractérisée en ce que ladite membrane comprend de plus au moins une couche additionnelle d'une matière polymère biocompatible.

5. Microcapsule selon la revendication 4, caractérisée en ce que la ou les couches additionnelles comprennent une matière polymère acrylique biocompatible extérieure contenant des groupes liés par covalence à une couche située sous la surface de ladite membrane.

6. Microcapsule selon l'une quelconque des revendications 1 à 5, caractérisée en ce que ledit polymère acrylique de matrice est polycationique et en ce que ledit polymère de membrane est polycationique et contient (a) des groupes ámines primaires et des groupes acides carboxyliques, (b) des groupes amines primaires et/ou secondaires ou (c) des groupes époxy et/ou des groupes amines tertiaires.

7. Microcapsule selon l'une quelconque des revendications 1 à 6, caractérisée en ce que ledit matériau cellulaire est constitué de cellules d'hybridome ou d'îlots de Langerhans.

8. Procédé pour former des microcapsules, caractérisé par (a) la formation d'une suspension aqueuse d'un matériau cellulaire vivant dans une solution aqueuse d'un premier polymère acrylique non toxique, biocompatible, soluble dans l'eau, qui est polyanionique ou polycationique et (b) l'introduction de ladite suspension aqueuse sous forme de gouttelettes dans une solution aqueuse d'un second polymère acrylique, non toxique, biocompatible, soluble dans l'eau, dont la charge est opposée à celle dudit premier polymère acrylique, de façon à provoquer une interaction entre les groupes anioniques et cationiques à l'interface entre la suspension de la matière vivante et la solution du second polymère acrylique et la formation d'un complexe polymère-polymère insoluble dans l'eau.

9. Procédé selon la revendication 8, caractérisé en ce que l'on fait réagir lesdites microcapsules avec un polymère additionnel contenant des groupes réagissant avec des groupes superficiels de la microcapsule, pour accroître l'épaisseur de la paroi de membrane microporeuse et pour conférer une résistance mécanique suffisante pour le maintien de la forme sphérique et de l'intégrité structurale lors de l'introduction in vivo.

10. Procédé selon la revendication 8 ou la revendication 9, caractérisé en ce que ladite suspension aqueuse et ladite solution aqueuse ont toutes deux un pH approximativement neutre et ledit permier et ledit second polymères acryliques sont présents sous forme d'un sel solubilisé.